# EUROPÄISCHE PATENTANMELDUNG

(11) **EP 4 120 027 A1**
(43) Veröffentlichungstag der Anmeldung: **18.01.2023**
(21) Anmeldenummer: 22182857.7
(22) Anmeldetag: 04.07.2022
(51) Int. Cl.: G03H 1/00, G01N 15/00, G01N 21/63, A61B 17/00, G02B 21/00, G02F 1/355, G02F 1/37, G03H 1/04, G03H 1/22, G01B 9/021

(54) **VERFAHREN ZUR POSITIONSBESTIMMUNG VON MIKRO- ODER NANOROBOTERN IN EINEM BIOLOGISCHEN GEWEBE, MIKRO- ODER NANOROBOTER SOWIE MESSANORDNUNG**

(30) Priorität: 13.07.2021 DE 102021118082
(71) Anmelder: Technische Universität Dresden, 01069 Dresden (DE)
(72) Erfinder: Czarske, Jürgen, 01062 Dresden (DE); Koukourakis, Nektarios, 01062 Dresden (DE)
(74) Vertreter: Kailuweit & Uhlemann Patentanwälte Partnerschaft mbB

(57) **Zusammenfassung**

Die Erfindung betrifft ein Verfahren zur Positionsbestimmung eines Mikro- oder Nanoroboters in einem biologischen Gewebe, bei dem ein an dem Mikro- oder Nanoroboter angeordneter nichtlinear optischer Kristall als Leitstern verwendet ist. Dabei wird das erzeugte, kohärente Licht halber Wellenlänge ausgewertet, wobei insbesondere die durch die Streuung im biologischen Gewebe resultierende Phasenverschiebung berücksichtigt wird. Die Streuinformationen können somit aufgrund der Kohärenz des erzeugten Lichtes berücksichtigt werden, was eine Fokussierung auf den nichtlinear optischen Kristall erlaubt. Außerdem kann eine spektrale Separation vom Hintergrundlicht ermöglicht werden. Es sind dabei sowohl räumlich als auch zeitlich hohe Auflösungen möglich. Die Erfindung betrifft auch einen Mikro- oder Nanoroboter mit einem nichtlinear optischen Kristall sowie eine Messanordnung zur Durchführung des Verfahrens, ein Computerprogrammprodukt und ein Datenverarbeitungssystem.

## Beschreibung

Die Erfindung betrifft ein Verfahren zur Positionsbestimmung, Verfolgung und Aktuierung von Mikro- oder Nanorobotern in einem biologischen Gewebe mittels einer optischen Methode unter Verwendung eines kohärenten Lichtstrahles, einen Mikro- oder Nanoroboter zum Einsatz in einem solchen Verfahren, die Verwendung eines Mikro- oder Nanoroboters in einem solchen Verfahren, eine Messanordnung zur Durchführung des Verfahrens, ein Computerprogrammprodukt zur Ausführung des Verfahrens sowie ein Datenverarbeitungssystem zur Durchführung des Verfahrens.

Medizinische Mikro- oder Nanoroboter haben großes Potenzial, einen Paradigmenwechsel in der Biomedizin herbeizuführen. Mithilfe medizinischer Mikromotoren ist es möglich, eine aktive Medikamentenverabreichung, eine Verbesserung der Mikrochirurgie oder eine Anwendung für künstliche Befruchtung auf minimalinvasive Weise zu realisieren.

Jedoch ist für eine große Anzahl von Anwendungen eine Echtzeitverfolgung und-steuerung der einzelnen Mikromotoren mit hoher räumlicher und zeitlicher Auflösung in tiefem Gewebe erforderlich.

### Stand der Technik

Es sind im Stand der Technik dafür bereits verschiedene Methoden bekannt. So können mittels Ultraschall Bilder auch tief im Gewebe erstellt und eine Echtzeit-Nachverfolgung durchgeführt werden. Nachteilig ist allerdings die unzulängliche räumliche Auflösung dieser Methode, was dazu führt, dass keine ausreichende räumliche Detektion der Objekte erfolgt. Alternativ wird auch die Magnetresonanztomografie eingesetzt. Dadurch ist man in der Lage eine Submillimeter-Auflösung zu gewährleisten, allerdings ist die zeitliche Auflösung aufgrund des aufwändigen Verfahrens unzureichend für eine In-situ-Detektion.

Eine weitere Möglichkeit für die Detektion der Mikro- oder Nanoroboter sind optische Methoden. Es wurden bereits Untersuchungen an mit Gold beschichteten, kugelförmigen Siliziumpartikeln, welche als konkav ausgebildete Spiegel fungieren, als Mikroreflektoren, durchgeführt. Es konnte dabei ein Signal durch 160 µm dickes Schädelgewebe realisiert werden. [1] Aufgrund eines zu geringen Signal-Rausch-Verhältnisses ist diese Methode allerdings auch unter Einsatz einer geeigneten Kamera sowie einer Erhöhung der Belichtungszeit in Verbindung mit einer Anpassung der Belichtungsintensität in Bezug auf die zu erreichende Tiefe stark limitiert.

Alternativ wurden bereits nichtlinear optische Kristalle als Marker für eine optische Detektion durch Gewebe genutzt. Dabei wurde kohärentes Laserlicht genutzt, welches in einem nichtlinear optischen Prozess eine anteilige Frequenzverdopplung verursacht, also zum Aussenden von Licht der halben Wellenlänge führt. Dadurch kann eine gute spektrale Separation vom Anregungslicht erreicht werden. Mittels dieser Methode konnte der nichtlinear optische Kristall durch bis zu 150 µm dickes Gewebe detektiert werden. Allerdings ist das Rauschen bei einer Durchstrahlung solch hoher Dicken dennoch sehr groß, was das Signal-Rausch-Verhältnis negativ beeinflusst und dazu führt, dass eine Anwendung dieser Methode für eine Detektion in tieferem Gewebe nicht möglich ist. [2] Optische Techniken haben dabei insbesondere den Nachteil, dass die Absorption der Strahlung und die starke Lichtstreuung bei zunehmender Tiefe zu einem unzureichenden Signalrauschverhältnis führen. Um die Absorption zu reduzieren, wird die Wellenlängenabhängigkeit dieses Prozesses ausgenutzt. So ist in den meisten der biologischen Gewebe die Absorption im roten und infraroten Bereich sehr gering und es überwiegt dann der Prozess der Streuung. [3]

Wenn ein Bündel kohärenten Lichtes streuendes Gewebe durchquert, so wird bei der Detektion statt eines Punktes oder Flecks durch den Prozess der Streuung ein Specklemuster erzeugt. Das Specklemuster ergibt sich durch die vielen einzelnen Streuprozesse innerhalb des Gewebes, an denen die Photonen beteiligt sind. Ein kohärenter Lichtstrahl erfährt eine große Anzahl von Streuprozessen innerhalb eines Materials. Das führt dazu, dass ein aus einer punktförmigen Lichtquelle ausgesendeter Lichtstrahl mit kleinem Strahldurchmesser nach dem Durchqueren eines Materials nicht als ein Punkt, sondern als Specklemuster erscheint. Dieser Effekt kann mittels eines Wellenfrontmodulators (SLM) korrigiert werden. Diese Wellenfrontmodulatoren sind Bauelemente, welche die Variablen, also die Phase und/ oder die Polarisation und/ oder die Amplitude, einer Lichtwelle gezielt verändern können. Die Variablen der Lichtwelle werden dabei spatiotemporal, also sowohl räumlich als auch zeitlich, manipuliert. Im Sinne der Erfindung ist eine Lichtwelle ein Lichtfeld. Es ist bei einem streuenden Medium oftmals ungünstig von einem Lichtstrahl auszugehen (Strahlenmodell) oder das Photonenmodell zu nutzen. Besser ist es hier das Licht als Lichtfeld beziehungsweise als Lichtwelle anzusehen, um die Ausbreitung des Lichtes in einem streuenden Medium zu beschreiben und zu manipulieren.

Die Korrektur des aufgrund der Streuung im Material auftretenden Specklemusters ist möglich, da bei der Streuung in den meisten biologischen Geweben die Informationen zwar verschlüsselt werden, aber nicht verloren sind. Dies bietet die Möglichkeit der Rekonstruktion des Bildes aus dem gestreuten Licht. In den letzten Jahren sind dazu eine Reihe von Techniken entwickelt worden, um dieses Ziel zu erreichen. Eine der effizientesten Methoden dafür ist die holographische Phasenkonjugation. Durch eine Anwendung der holografischen Phasenkonjugation kann ein Fokus durch die streuenden Medien wiederhergestellt werden, wenn ein Leitstern an der Stelle vorhanden ist, an der der Fokus erzeugt werden soll. Die harmonische Holographie führt dabei einen einzigartigen nichtlinear optischen, kontrastbildenden Mechanismus ein, nämlich die Erzeugung der zweiten Harmonischen (SHG). Dadurch konnten Muster im Gewebe erfasst werden. Die erreichte Tiefe lag dabei bisher nur bei ca. 100 µm. [4]

Zur Korrektur der Streuprozesse wird eine geeignete Beugungsmaske zur Korrektur der Streueffekte durch einen Wellenfrontmodulator (SLM) zur Verfügung gestellt. Um eine dafür geeignete Maske zu erzeugen, werden iterative Prozesse genutzt, was allerdings mehrere Messprozesse und somit nachteilig eine relativ lange Zeit benötigt. Da sich das Gewebe in der Regel bewegt, ist hier ein besonders schneller Prozess notwendig.

### Aufgabe der Erfindung

Es wird eine Methode zur Positionsbestimmung und der Aktuierung bzw. Manipulation, wie der Aufheizung, Bewegung, Transfektion, Lasermaterialbearbeitung der Mikro- oder Nano-Roboter gesucht, welche die Nachteile aus dem Stand der Technik überwindet und insbesondere eine Bestimmung der Position mit hoher zeitlicher und räumlicher Auflösung auch in tieferem Gewebe erlaubt.

### Lösung der Aufgabe

Erfindungsgemäß wird die Aufgabe durch ein Verfahren, einen Mikro- oder Nanoroboter, eine Verwendung, eine Messanordnung, ein Computerprogrammprodukt sowie ein Datenverarbeitungssystem gemäß den unabhängigen Ansprüchen gelöst. Vorteilhafte Ausgestaltungen der Erfindung sind in den abhängigen Ansprüchen angegeben.

Die Aufgabe wird insbesondere durch ein Verfahren zur Positionsbestimmung von Mikro- oder Nanorobotern in einem biologischen Gewebe gelöst, welches folgende Schritte umfasst:
**1)** Aussenden eines gepulsten Laserstrahls mit einer Wellenlänge λ.
**2)** Teilen des gepulsten Laserstrahls in einen kohärenten Messstrahl der Wellenlänge λ und einen kohärenten Referenzstrahl der Wellenlänge λ
**3)** Leiten des kohärenten Messstrahles auf einen in dem biologischen Gewebe angeordneten Mikro- oder Nanoroboter mit wenigstens einem nichtlinear optischen Kristall und Leiten des kohärenten Referenzstrahles auf einen nichtlinear optischen Referenzkristall. Der Mikro- oder Nanoroboter weist dabei wenigstens einen nichtlinear optischen Kristall auf.
**4)** Überlagerung des durch den nichtlinear optischen Referenzkristall unter Erzeugung der zweiten Harmonischen emittierten, kohärenten Referenzstrahlbündels der Wellenlänge λ/2 mit dem durch den nichtlinear optischen Kristall des Mikro- oder Nanoroboters unter Erzeugung der zweiten Harmonischen emittierten, kohärenten Messstrahlbündels der Wellenlänge λ/2
**5)** Detektion des zweidimensionalen Interferenzmusters der Überlagerung aus dem Referenzstrahlbündel und dem Messstrahlbündel, wobei aus dem Interferenzmuster ein Phasenmuster des oder der Messstrahlenbündel gegenüber dem Referenzstrahlbündel ermittelt wird. Es wird hier mittels der Auswertung des zweidimensionalen Interferenzmusters nach dem Prinzip eines digitalen Hologramms die Amplituden- und / oder die Phaseninformation der gestreuten Welle ermittelt. Insbesondere wird hier die Phaseninformation genutzt.
**6)** Ermittlung der Position des Mikro- oder Nanoroboters. Dabei wird nach einer möglichen Ausführungsform ein kohärentes Messstrahlenbündel mit einer Wellenlänge λ/2 mittels eines aus dem Interferenzmuster ermittelten Phasenverschiebungsmusters korrigiert und auf den Mikro- oder Nanoroboter geleitet und das reflektierte Licht mittels einer zweiten Detektionseinheit detektiert. Dabei wird bevorzugt eine statistische Auswertung genutzt, beispielsweise unter Verwendung eines neuronalen Netzes. Es wird dabei die Bestimmung der lateralen Position durchgeführt, vorzugsweise wird auch die Tiefe der Position im Gewebe ermittelt.

Die laterale Information über Verschiebungen des Mikro- und Nanoroboters ist in der Intensitätsverteilung des reflektierten SHG-Wellenfeldes enthalten. Dabei wird eine Quasi-Punktquelle, SHG-nichtlinear optisches Kristall im Roboter, und makroskopisch homogenes Gewebe angenommen. Eine absolute Position kann aus statistischen Verschiebungsmessungen ermittelt werden.

Da das Gewebe unbekannt ist, kann man die Homogenität durch die Wahl kürzerer Pulse unter Beachtung der Kohärenzlänge und unter Betrachtung des Interferenzmusters abschätzen. Für die axiale Position wird bevorzugt die Double Helix-Methode genutzt, da die Tiefe, also die axiale Position, mit einem sich drehenden Intensitätsmuster abschätzbar ist.

Zur Navigation und zum Tracking des Mikro- oder Nanoroboters kann ein geschlossener Regelkreis zur Anwendung kommen, wobei die Zeit zur Ermittlung der Position vorteilhaft im Bereich von ca. 10 ms realisiert werden kann. Das Tracking ist im Sinne der Erfindung eine zeitlich aufeinanderfolgende Folge von Positionsbestimmungen.

Das gepulste Laserlicht weist nach einer vorteilhaften Variante Femtosekundenpulse auf. Die Pulse haben also eine Dauer kleiner als 1 ps, vorteilhaft im Bereich von ca. 100 fs.

Kurze Pulse weisen vorteilhaft entsprechend große Pulsleistungen auf, die für die Konversionseffizienz entscheidend sind. Die Pulsleistung ist besonders relevant, da die SHG-Leistung proportional zum Quadrat der Pulsleistung ist.

Ultrakurze Pulse weisen weiterhin vorteilhaft keine nennenswerte thermische Erwärmung des Gewebes auf. Es kommt dabei nicht zu einer Thermalisierung, wenn die Zeit der Anregung durch den Laserpuls kürzer ist als die Relaxationszeit.

Die Detektion des Interferenzmusters nach Schritt 5 erfolgt mit einer ersten Detektionseinheit, welche bevorzugt als Kamera ausgebildet ist.

Eine mögliche Ausgestaltung des Verfahrens sieht vor, eine Manipulation des Mikro- oder Nanoroboters, also eine gezielte Bewegung, mit der Positionsbestimmung des Mikro- oder Nanoroboters zu kombinieren und so eine Kombination aus Steuerung und Echtzeit-Tracking des Mikro- oder Nanoroboters zu ermöglichen. Zur Manipulation der Position des Mikro- oder Nanoroboters wird dabei bevorzugt ein äußeres Magnetfeld genutzt, welches auf ein am Mikro-oder Nanoroboter angeordnetes Aktormaterialelement einwirkt. Das Aktormaterialelement wirkt dabei als Angriffspunkt für eine Kraft zur Bewegung und ist bevorzugt aus Metall oder einem ferromagnetischen Material ausgebildet.

Das Verfahren umfasst dann also den Schritt der gezielten Positionsänderung des Mikro- oder Nanoroboters und der nachfolgenden Positionsbestimmung nach Schritt 6). Um ein Tracking zu erhalten werden die einzelnen Schritte dabei wiederholt durchgeführt.

Dabei wird die Position wiederholt bestimmt, um so eine nahezu kontinuierliche Positionsbestimmung zu ermöglichen und eine Trajektorie aufzunehmen.

Eine mögliche Variante sieht dabei vor, dass bereits der kohärente Messstrahl während des Schrittes 3) über einen Wellenfrontmodulator geleitet wird, so dass eine möglichst gute Fokussierung auf den als Leitstern wirkenden nichtlinear optischen Kristall erfolgt. Dafür muss vorher eine entsprechende Messung durchgeführt werden, um die Phasenmaske für den Wellenfrontmodulator bereitstellen zu können.

Bevorzugt wird die Ermittlung der Position des Mikro- oder Nanoroboters gemäß Schritt 6) folgende Schritte umfassend ausgeführt:
6.1) Ermittlung eines zweidimensionalen inversen Phasenverschiebungsmusters aus dem zweidimensionalen Interferenzmuster mittels digital optischer Holographie. Es wird die durch die Streueffekte im biologischen Gewebe auftretende Phasenverschiebung ermittelt, um sie dann korrigieren zu können. Dabei wird das Phasenverschiebungsmuster des durch den als Leitstern wirkenden nichtlinear optischen Kristall des Mikro- oder Nanoroboters emittierten Messstrahlbündels der Wellenlänge λ/2 gegenüber dem durch den nichtlinear optischen Referenzkristall emittierten Referenzstrahlbündels der Wellenlänge λ/2 aus dem zweidimensionalen Interferenzmuster mittels digital optischer Holographie ermittelt.
6.2) Leiten eines kohärenten Messstrahlenbündels auf einen das inverse Phasenverschiebungsmuster aufweisenden Wellenfrontmodulator, wodurch das kohärente Messstrahlenbündel eine Korrektur in Form einer digital-optischen Phasenkonjugation erfährt
6.3) Leiten des korrigierten Messstrahlenbündels auf den Mikro- oder Nanoroboter mit dem nichtlinear optischen Kristall
6.4) Aufnahme eines Intensitätsmusters des durch den Mikro- oder Nanoroboter reflektierten Lichts mittels einer zweiten Detektionseinheit
6.5) Bestimmung der lateralen Position des Mikro- oder Nanoroboters aus dem aufgenommenen Intensitätsmuster

Diese Ausführungsvariante wird auch als sendeseitiges Beamforming bezeichnet.

Eine Bestimmung der Position des Mikro- oder Nanoroboters erfolgt mit der Aufnahme, bevorzugt einer Kameraaufnahme, des Streulichts vom mit dem Wellenfrontmodulator beleuchteten Mikro- oder Nanoroboter durch die zweite Detektionseinheit, welche vorzugsweise eine Kamera ist. Die laterale Position kann durch eine Schwerpunktbildung des Intensitätsmusters bestimmt werden. Nach einer möglichen Ausgestaltung kann die zweite Detektionseinheit auch als eine Photodiode, also eine 1-Pixel-Kamera, ausgebildet sein.

Eine bevorzugte Variante des Verfahrens sieht vor, dass mittels einer Regelung die Ermittlung des inversen Phasenverschiebungsmusters nach Schritt 6.1) und die Wellenfrontformung des kohärenten Lichtstrahles mittels des Wellenfrontmodulators als selbstlernendes System ausgebildet sind. Hierfür kann ein Echtzeitsystem für die Regelung eingesetzt werden. [5]

Daraus ergibt sich ein Tracking des Mikro- oder Nanoroboters. Tracking ist im Sinne der Erfindung eine zeitlich aufeinanderfolgende, wiederholte Positionsbestimmung. Aufgrund der Möglichkeit eine sehr schnelle Positionsbestimmung durchführen zu können, kann hier vorteilhaft ein Echtzeit-Tracking durchgeführt werden. Dabei wird nach einer möglichen Variante die laterale, also zweidimensionale, Position des Mikro- oder Nanoroboters in Echtzeit bestimmt und nach einer bevorzugten Variante wird eine dreidimensionale Positionsbestimmung und somit ein dreidimensionales Tracking durchgeführt. Echtzeit bedeutet im Sinne der Erfindung innerhalb einer kurzen Zeit, die garantiert wird, bevorzugt weniger als ca. 10 ms.

Vorteilhaft kann dabei eine Kalibration zur Verbesserung des Signal-Rausch-Verhältnisses beitragen.

Bevorzugt wird auch die Tiefe der Position des Mikro- oder Nanoroboters ermittelt, wodurch dann die dreidimensionale Position des Mikro- oder Nanoroboters bestimmt werden kann. Um die Tiefeninformation zu erhalten können unterschiedliche Kodierungsverfahren, wie beispielsweise eines unter Verwendung von Doppelhelixwellen, genutzt werden. [6]

Kohärentes Licht bezeichnet die Eigenschaft von Wellen, dass es zwischen zwei Wellenzügen eine feste Phasenbeziehung gibt. Nur wenn die Phasenbeziehung zwischen den beiden Wellenzügen nahezu konstant bleibt, ist die Entstehung eines stabilen Interferenzmusters möglich. Dabei ist im Sinne der Erfindung Licht auch dann kohärent, wenn dessen Kohärenzlänge wenigstens dem auftretenden Gangunterschied bei der Streuung im biologischen Gewebe entspricht.

Ein nichtlinear optischer Kristall ist im Sinne der Erfindung ein nicht-zentrosymmetrisches Medium, insbesondere ein Kristall mit einer großen Nichtlinearität, wie beispielsweise Bariumtitanat (BTO oder BaTiO₃) oder Bariumborat (BBO). Auch der SHG-Koeffizient sowie eine ausreichende Biokompatibilität sind relevante Faktoren für die Auswahl eines geeigneten Materials.

Grundsätzlich sind als nichtlinear optische Kristalle nichtinversionssymmetrische Materialien erforderlich, welche auch eine große Bandlücke aufweisen müssen, also in den relevanten Wellenlängenbereichen transparent sein müssen, wie beispielsweise BTO, BBO, KnbO₃, LiNbO₃ oder auch organische Materialien oder auch SHG-aktive kristalline organisch-anorganische Hybridnanopartikel. Dabei zeichnet sich BTO besonders durch eine gute Biokompatibilität aus.

In nichtlinear optischen Kristallen kann der nichtlinear optische Effekt der Erzeugung einer zweiten Harmonischen bei der doppelten Frequenz, also der halben Wellenlänge, beobachtet werden. Im Gegensatz zum Effekt der Frequenzverdopplung behält bei der Erzeugung der zweiten Harmonischen innerhalb eines nichtlinear optischen Kristalls die emittierte Strahlung der halben Wellenlänge eine deterministische Phasenbeziehung zum Grundfeld und ist somit eine kohärente Welle. Dabei ist der Prozess der Erzeugung der zweiten Harmonischen nur effizient in nicht zentrosymmetrischen, kristallinen Strukturen, da die Symmetrie gebrochen wird. Da viele Materialien dafür nicht geeignet sind, können auch spezielle Nanokristalle innerhalb von Materialien als Marker verwendet werden, wie beispielsweise BaTiO₃ (BTO), BBO, ZnO, Fe(IO₃)₃, KNbO₃, KTiOPO₄ (KTP) CdSe, CdS, CdTe, GaAs, InP.

Aufgrund der Phasenkohärenz der zweiten Harmonischen wird hier eine instantane Phasenmessung durchgeführt, was für eine Echtzeitsignalverarbeitung beziehungsweise eine Messung in Echtzeit vorteilhaft ist.

Um den wellenlängenabhängigen Absorptionseigenschaften der biologischen Gewebe Rechnung zu tragen werden für den Anregungsstrahl bevorzugt eine Wellenlänge im infraroten Bereich, insbesondere im nahinfraroten Bereich, genutzt, wobei besonders bevorzugt auch die zweite Harmonische bei λ/2 noch in einem geeigneten Bereich, bei dem wenig absorbiert wird. Das ist insbesondere der nahinfrarote Bereich.

Eine Emission des Lichtes der zweiten Harmonischen kann dabei in Abhängigkeit von den doppelbrechenden Eigenschaften des nichtlinear optischen Kristalls in verschiedene Richtungen erfolgen. Das hier betrachtete Messstrahlenbündel der Wellenlänge λ/2 ist dabei im Sinne der Erfindung ein Ausschnitt aus dem emittierten Licht, wobei gerade der Teil des emittierten Lichtes betrachtet wird, welcher auf die Detektionseinheit (Kamera) trifft. Bevorzugt wird das Signal der zweiten Harmonischen in dem nichtlinear optischen Kristall des Mikro- oder Nanoroboters in Reflektion, also entgegengesetzt zur Anregungsrichtung betrachtet. Für den Referenzkristall kann auch das Signal in Anregungsrichtung, also entsprechend der Transmissionsrichtung genutzt werden.

Für einen effizienten Prozess der Erzeugung der zweiten Harmonischen sind hohe Anregungsintensitäten notwendig, da die erzeugten Signale sonst zu gering sind. Eine Anregung wird somit bevorzugt durch gepulste Laser generiert.

Der Referenzstrahl und der Messstrahl werden in einer Anordnung nach der Art eines Interferometers geleitet. Aus dem Interferenzmuster der beiden überlagerten Strahlen kann eine Information bezüglich der Phasenverschiebung des Messstrahles durch das Gewebe ermittelt werden. Die Lauflängen, also die optischen Weglängen unter Berücksichtigung der Brechungsindizes der Materialien, zwischen dem Messstrahl und dem Referenzstrahl müssen dabei übereinstimmen. Abweichungen innerhalb der Kohärenzlänge können toleriert werden.

Ein biologisches Gewebe ist im Sinne der Erfindung ein inhomogenes, lichtstreuendes Medium, in dem differenzierte Zellen mit einer extrazellulären Matrix vorliegen.

Ein Mikro- oder Nanoroboter ist im Sinne der Erfindung ein Bauelement, dessen größte Ausdehnung im Bereich von 500 nm bis 500 µm, insbesondere im Bereich 10-40 µm oder 10-20 µm, liegt. Dabei werden Mikro- oder Nanoroboter entweder aktiv oder passiv betrieben. Aktive Mikro- oder Nanoroboter weisen dabei interne Elemente zur Steuerung auf. Passive Mikro- oder Nanoroboter werden extern durch äußere, also außerhalb des Mikro- oder Nanoroboters angeordnete, Stimuli gesteuert. Möglich ist dabei eine Steuerung aufgrund eines gezielt erzeugten magnetischen Feldes, welches eine Kraft auf innerhalb des Mikro- oder Nanoroboters angeordnete Magneten oder magnetisierbare Schichten ausübt.

Mikro- oder Nanoroboter können in der Medizin vielfältig eingesetzt werden. Im menschlichen Organismus können beispielsweise Krebszellen minimal invasiv diagnostiziert und therapiert werden. In der Diagnostik können sie mechanische oder biochemische Informationen ermitteln und damit Zellen oder Gewebe überwachen. Patienten können gezielt Medikamente zugeführt werden oder Bakterienbereiche durch Erhitzung zerstört werden.

Ein kohärenter Lichtstrahl erfährt eine große Anzahl von Streuprozessen innerhalb eines Materials. Das führt dazu, dass ein aus einer punktförmigen Lichtquelle ausgesendeter Lichtstrahl mit kleinem Strahldurchmesser nach dem Durchqueren eines Materials nicht als ein Punkt, sondern als Specklemuster erscheint. Dieser Effekt kann mittels eines Wellenfrontmodulators (SLM) korrigiert werden. Dafür muss zunächst die Phasenstörung des Lichtes aufgrund der Streueffekte ermittelt werden und danach ein kohärenter Lichtstrahl mit einer entsprechend invertierten Information versehen werden. Das wird durch ein durch den Wellenfrontmodulator vorgegebenes Beugungsmuster realisiert. Wenn nun also nach der Konzeption der Erfindung unter Verwendung des Verfahrens der Wellenfrontformung mittels eines SLM ein entsprechend modulierter Lichtstrahl auf einen an oder in dem Mikro- oder Nanoroboter angeordneten nichtlinear optischen Kristall trifft, so kann das reflektierte Licht mit halber Wellenlänge entsprechend dem Konzept eines Leitsterns detektiert werden. Es kann durch die Verwendung des Verfahrens der Wellenfrontformung somit ein gutes Signal-Rausch-Verhältnis auch bei einer großen Anzahl an Streuprozessen erreicht werden. Somit können Positionsmessungen in deutlich größeren Tiefen vorgenommen werden.

Die Besonderheit des Verfahrens liegt hier darin, dass der reflektierte Lichtstrahl ebenfalls kohärent ist, sodass die Phaseninformation im Gegensatz zu den Verfahren aus dem Stand der Technik nicht verloren geht. Dies ermöglicht eine Wellenfrontformung unter gleichzeitiger Ausnutzung der spektralen Separation des reflektierten Signals.

Übliche digitale Holographie erlaubt zwar 3D-Positionsmessungen, hier wird aber erfindungsgemäß unter Nutzung der Aberrationskorrektur durch ein streuendes Medium gemessen, so dass eine weitere statistische Auswertung erforderlich ist. Demgegenüber kann die Aktuatorik, also das Aufheizen des Mikro- oder Nanoroboters, unmittelbar vorgenommen werden.

Die digital-optische Holographie (digital-optische Phasenkonjugation) hat dabei den Vorteil, dass sie keine zeitaufwendigen Iterationen oder zeitraubende Kalibrierungsmessungen erfordert, sondern eine direkte Wellenfrontformung mit einer einzigen Messung ermöglicht.

In dem Verfahren wird aus dem zweidimensionalen Phasenverschiebungsmuster unter Verwendung der digital-optischen Holographie eine Beugungsmaske ermittelt und ein auf einen Wellenfrontmodulator, der diese Beugungsmaske aufweist, auftreffender kohärenter Lichtstrahl erfährt eine Wellenfrontformung in Form einer digital-optischen Phasenkonjugation, sodass der kohärente Lichtstrahl eine zu dem ermittelten, zweidimensionalen Phasenverschiebungsmuster invertierte Phasenverschiebung erhält und dieser Lichtstrahl wird mit der invertierten Phasenverschiebung für weitere Positionsbestimmungsmessungen verwendet.

Für eine Wellenfrontformung mit einem Wellenfrontmodulator wird das inverse Phasenverschiebungsmuster genutzt. Es liegt eine digitale optische Phasenkonjugation vor, die zu einer Zeitumkehr der Lichtwellenausbreitung führt.

Die Methode, bei der Leitsterne genutzt werden und eine quantitative Auswertung der Phase des gestreuten Lichtes durchgeführt wird, wurde bereits zur transmittalen Untersuchung von Mäusegewebe genutzt. [7] Dabei wurde festgestellt, dass das mittels eines SLM korrigierte Signal räumlich entlang des Gewebes im Mikrometerbereich verschoben werden kann, um noch einen geeigneten verschobenen, rekonstruierten Fokus zu erhalten.

Für die digital-optische Phasenkonjugation werden also der oder die nichtlinear optischen Kristalle als Leitsterne verwendet, um die Information zu extrahieren. Die Phase des Leitsternlichts wird dabei durch quantitative Phasenmessungen, durch digitale Holographie oder andere Methoden, wie beispielsweise neuronale Netze, erfasst und es wird eine Phasenmaske in Form einer Beugungsmaske der Phasenkonjugierten auf dem Wellenfrontmodulator dargestellt. Dieser Ansatz ermöglicht es, die Streuprozesse zeitlich umzukehren und den Leitstern neu zu erzeugen.

Bei der digitalen Phasenkonjugation ist vorteilhaft eine einzelne Messung ausreichend, um eine geeignete Maske zu erstellen. Es kann die Phase des gestreuten Lichtes dabei derart korrigiert werden, als wäre dies ein zeitumkehrender Effekt, welcher die Fokussierung selbst bei einer Transmission durch 400 µm dickes Maus-Schädelgewebe erlaubt. [8]

Nach einer möglichen Variante wird in Schritt 6.2) ein durch den Referenzkristall ausgesandtes, kohärentes Lichtstrahlenbündel der Wellenlänge λ/2 verwendet und das Signal des durch den nichtlinear optischen Kristall elastisch gestreuten Lichtstrahlbündels der Wellenlänge λ/2 wird detektiert. Dabei wird ein kohärentes Lichtstrahlenbündel der Wellenlänge λ/2 genutzt, dessen Wellenfront durch den Wellenfrontmodulator eine Modulation, also eine Phasenkonjugation erfährt und so trotz Streuung auf den Mikro- oder Nanoroboter fokussierbar ist. Dabei weist die Phasenmaske des Wellenfrontmodulators (Beugungsmaske) die konjugierte Phase des aus dem Interferenzmuster bestimmten Hologramms auf.

Eine alternative Variante des Verfahrens sieht vor für die Ermittlung des zweidimensionalen Phasenverschiebungsmusters nach Schritt 6.1) eine Transformation auf ein Phasenverschiebungsmuster für die Ursprungswellenlänge λ zu enthalten. Dabei kann in Schritt 6.2) ein kohärenter Lichtstrahl der Wellenlänge λ verwendet werden. Dabei wird ein zweidimensionales Phasenverschiebungsmuster des durch den nichtlinear optischen Kristall des Mikro- oder Nanoroboters emittierten Messstrahlbündels der Wellenlänge λ/2 gegenüber dem durch den nichtlinear optischen Referenzkristall emittierten Referenzstrahlbündels der Wellenlänge λ/2 aus dem mittels einer Detektionseinheit aufgenommenen, zweidimensionalen Interferenzmuster mittels digital optischer Holographie ermittelt.

Nach dieser möglichen Variante wird also ein kohärenter Lichtstrahl der Wellenlänge λ genutzt, dessen Wellenfront durch den Wellenfrontmodulator eine Modulation erfährt. Dabei muss das Phasenverschiebungsmuster, welches mittels kohärenten Lichtes der Wellenlänge λ/2 erzeugt wurde, für eine entsprechende Beugungsmaske für einen kohärenten Lichtstrahl der Wellenlänge λ angepasst werden. Mittels der angepassten Beugungsmaske ist die Streuung im Gewebe korrigierbar und der kohärente Lichtstrahl der Wellenlänge λ ist auf den Mikro- oder Nanoroboter fokussierbar.

Eine spezielle Variante des erfindungsgemäßen Verfahrens, das sogenannte empfangsseitige Beamforming, sieht vor, dass die Schritte 3) bis 5) mehrmals nacheinander durchgeführt werden, wobei der kohärente Messstrahl in Schritt 3) jeweils in einem unterschiedlichen, eingestellten Einfallswinkel auf den nichtlinear-optischen Kristall des Mikro- oder Nanoroboters fällt. Daraus kann die Position gemäß Schritt 6) ermittelt werden.

Nach einer möglichen Variante werden dabei die Schritte 3) bis 5) gleichzeitig durchgeführt. Das wird dann mehrmals wiederholt. Dadurch können vorteilhaft in-vivo Untersuchungen mit strömendem Blut durchgeführt werden, da dies ein Echtzeit-Tracking mit geringer Latenz von teilweise unter 1 ms erlaubt. Bevorzugt wird das in einer closed-loop umgesetzt.

Alternativ werden die Schritte 3) bis 5) nacheinander durchgeführt und das wird dann mehrmals wiederholt.

Nach einer möglichen Variante erfolgt die Ermittlung der Position nach Schritt 6) unter Auswertung unterschiedlicher aus den Interferenzmustern ermittelter Phasenverschiebungsmuster mittels neuronaler Netze. Diese werden nach einer möglichen Variante bereits vorher trainiert. So kann eine Positionsbestimmung auch aus einer einzelnen Messung erfolgen.

Bevorzugt wird dabei die Einstellung der Einfallswinkel dadurch realisiert, dass der kohärente Messstrahl durch einen zeitlich modulierten Wellenfrontmodulator oder einen rotierenden Spiegel, insbesondere ein Galvospiegel, oder ein Scanner in seinem Verlauf beeinflusst wird.

Dabei wird eine inkrementelle Auswertung vorgenommen, bei der die einzelnen Wellenberge im Verlauf ausgewertet werden.

Eine besonders vorteilhafte Variante des Verfahrens sieht vor, dass der Mikro- oder Nanoroboter mittels eines externen magnetischen Feldes in Rotation mit einer Rotationsfrequenz versetzt wird, sodass das in Schritt x detektierte, auszuwertende Signal mit der Rotationsfrequenz oder mit der doppelten Rotationsfrequenz moduliert ist

Der Mikroroboter weist dafür beispielsweise Metallpartikel oder einen Magneten auf und kann dann mittels des Magnetfeldes, beispielsweise hervorgerufen durch Helmholtzspulen, in Rotation versetzt werden, sodass eine Auswertung des reflektierten Signals auf Basis der Rotationsfrequenz vorgenommen werden kann. Das relevante Signal kann dadurch effektiv von zusätzlichen Effekten, wie beispielsweise solchen, die durch Kollagen verursacht werden, separiert werden. Für die Auswertung wird dabei insbesondere ein Lock-In-Verstärker verwendet.

Im Kollagen der Haut findet ein Bruch der Innversionssymmetrie statt, so dass SHG auftritt. Hierdurch entsteht ein SHG-Hintergrundsignal, welches mit weiteren Methoden zu separieren ist. Die gezielte Bewegung und Rotation des Mikro- oder Nanoroboters in Verbindung mit Lock-in-Heterodyning, also einer frequenzspezifischen Auswertung, verbessert hier das Signal-Rausch-Verhältnis ganz erheblich.

Dabei werden die doppelbrechenden Eigenschaften eines nichtlinear optischen Kristalls ausgenutzt. Tritt ein Strahl in einen doppelbrechenden Kristall ein, so wird er in zwei Strahlen aufgespalten: den ordentlichen und den außerordentlichen Strahl. Im ordentlichen Strahl ist das elektrische Feld senkrecht zu der Ebene polarisiert, die durch die Ausbreitungsrichtung und die Kristallachse aufgespannt wird. Im außerordentlichen Strahl ist das elektrische Feld parallel zu dieser Ebene polarisiert. Aufgrund der daraus resultierenden Richtungsabhängigkeit des Streuquerschnittes des nichtlinear optischen Kristalls und somit des Signals, schwankt das Signal somit mit der doppelten Rotationsfrequenz. Wenn der Mikro- oder Nanoroboter zu einer Seite abgeschattet ausgebildet ist, so ist das resultierende Signal mit der Rotationsfrequenz moduliert. Es können Schwankungen des Signals von einer Größenordnung, also einem Faktor 10, beobachtet werden und somit kann durch eine frequenzabhängige Auswertung des Signals das Signal-Rausch-Verhältnis weiter verbessert werden. Durch eine magnetisch aktuierte Rotation des Mikro- oder Nanoroboters kann eine zeitliche Modulation für eine verbesserte Trennschärfe genutzt werden. Mittels einer frequenzabhängigen Auswertung ist es möglichauch eine Trennung der Signale, welche durch Gewebe Lichtstreuung verursacht ist, oder jene, welche durch den rotierenden Mikroroboter reflektiert werden, zu erreichen. Ein gedrehter Mikro- oder Nanoroboter kann somit als blinkend betrachtet und dadurch besser detektiert werden.

Die Positionsmessung umfasst nach einer vorteilhaften Variante folgende Schritte:
a) Fokussieren eines kohärenten Lichtstrahles auf einen Punkt (x, y, z) im biologischen Gewebe,
b) Beleuchtung des biologischen Gewebes an der Position (x, y, z) mit dem Strahl kohärenten Lichtes,
c) Detektion der durch den nichtlinear optischen Kristall erzeugten zweiten Harmonischen,
d) Wiederholung der Schritte a), b), c) an unterschiedlichen Positionen, sodass ein Intensitätsprofil für die unterschiedlichen Positionen erstellt wird und
e) Ermittlung der Position des Intensitätsmaximums.

Die Position wird also mittels rastern des Bereiches bestimmt.

Eine mögliche Variante des Verfahrens sieht vor, dass eine spektrale Filterung zur Separation des zu detektierenden Lichts, insbesondere des zu detektierenden Messstrahlenbündels, der halben Wellenlänge λ/2 von dem Hintergrundlicht mit der Wellenlänge λ durch einen zwischen der Detektionseinheit und dem biologischen Gewebe angeordneten spektralen Filter erfolgt.

Bevorzugt wird der korrigierte Messstrahl oder das korrigierte Messstrahlenbündel gemäß Schritt 6.3) auf den Mikro- oder Nanoroboter fokussiert, sodass eine gezielte Steuerung der Temperatur des Mikro- oder Nanoroboters durch eine Variation der Intensität des auf den Mikro- oder Nanoroboter fokussierten, korrigierten Messstrahles oder korrigierten Messstrahlenbündels erfolgt. Eine mögliche Variante des Verfahrens sieht ganz allgemein eine Erhitzung des Mikro-oder Nanoroboters durch eine Variation der Intensität des eingestrahlten Lichtes vor. Dadurch kann auch eine möglich temperaturinduzierte Freigabe eines Stoffes erfolgen. Es ist dabei insbesondere eine Erwärmung von im nichtlinear optischen Kristall angeordneten Metallen möglich.

Dabei wird aus dem zweidimensionalen Phasenverschiebungsmuster unter Verwendung der digital-optischen Holographie eine Beugungsmaske ermittelt und ein auf einen Wellenfrontmodulator, der diese Beugungsmaske aufweist, auftreffender kohärenter Lichtstrahl erfährt eine Wellenfrontformung in Form einer digital-optische Phasenkonjugation, sodass der kohärente Lichtstrahl eine zu dem ermittelten zweidimensionalen Phasenverschiebungsmuster invertierte Phasenverschiebung erhält und dass dieser kohärente Lichtstrahl mit der invertierten Phasenverschiebung auf den Mikro- oder Nanoroboter fokussiert wird, sodass eine gezielte Steuerung der Temperatur des Mikro- oder Nanoroboters durch eine Variation der Intensität des auf den Mikro- oder Nanoroboter fokussierten Lichtstrahls erfolgt.

Eine lokale Erwärmung des Gewebes kann eine direkte Einwirkung auf die umgebenden Zellen, beispielsweise Krebszellen, bewirken. Durch Zellablation können dadurch Krebszellen zerstört werden oder Bakterieninfektionen bei einer Aufheizung auf über ca. 60 °C behandelt werden.

Besonders bevorzugt umfasst das Verfahren einen Schritt, bei dem eine temperaturabhängige Freigabe eines Stoffes erfolgt. Eine Alternative ist die Transfektion mittels Lasermaterialbearbeitung. Ein solcher Stoff ist beispielsweise ein Medikament zur Diagnostik.

Dabei kann nach einer vorteilhaften Variante eine Erwärmung von Metallpartikeln im nichtlinear optischen Kristall erfolgen. Dadurch können verschiedene Effekte erzielt werden. Eine aufgrund des photo-thermischen Effektes erfolgte Erwärmung des nichtlinear optischen Kristalls kann beispielsweise für eine Medikamentenabgabe genutzt werden. Eine lokale Erwärmung des Gewebes kann auch direkt Krebszellen durch Zellablation zerstören oder Bakterieninfektionen bei der Aufheizung über ca. 60 °C behandeln [9]. Dafür kann ein Verfahren mit Chirp-Pulsverbreiterung in einer Glasfaser genutzt werden. Dies ist notwendig, weil eine Temperaturerhöhung eines Festkörpergitters in der Regel einen Wärmetransfer von den durch Photonen aufgeheizten Elektronen auf die Phononen, also die Gitterschwingungen, erfordert. Daher sind für eine Thermalisierung lange Laserpulse erforderlich, die durch Pulsverbreiterung erzeugt werden.

Ein weiterer Aspekt der Erfindung betrifft einen Mikro- oder Nanoroboter zur Anwendung in biologischem Gewebe, aufweisend ein Aktormaterialelement als Angriffspunkt für eine Kraft zur Bewegung des Mikro- oder Nanoroboters. Der Mikro- oder Nanoroboter weist außerdem einen nichtlinear optischen Kristall als Leitstern auf. Der nichtlinear optische Kristall hat bevorzugt wenigstens eine Ausdehnung in wenigstens einer Dimension von 1 µm und besonders bevorzugt von wenigstens 2 µm oder 5 µm.

Nach einer vorteilhaften Ausgestaltung sind mehrere kleine nichtlinear optische Kristalle in dem Mikro- oder Nanoroboter angeordnet.

Ein solches Aktormaterialelement kann beispielsweise aus einem ferromagnetischen Material ausgebildet sein. Der Mikro- oder Nanoroboter ist dann durch den Stimulus eines externen Magnetfeldes passiv bewegbar. Alternativ kann das Aktormaterialelement auch durch eine innerhalb des Mikro- oder Nanoroboters erzeugte Kraft angeregt werden. Mikro- oder Nanoroboter können als Partikel in Form von Stäben, Rohren, Kugeln und Spiralen realisiert werden und durch chemische Reaktionen oder physikalische Felder, wie beispielsweise Magnetfelder, oder bewegliche Zellen bewegt werden. Die Funktionen reichen von minimalinvasiver Chirurgie bis zu einer gezielten Medikamentenverabreichung und Diagnostik.

Bevorzugt weist der Mikro- oder Nanoroboter ferner ein aus einem magnetischen oder einem ferromagnetischen Material ausgebildetes Element auf. Es ist also zusätzlich ein Magnet oder ein Ferromagnet an dem Mikro- oder Nanoroboter angeordnet.

Der nichtlinear optische Kristall ist nach einer vorteilhaften Ausgestaltung des Mikro- oder Nanoroboters aus Bariumtitanat (BaTiO₃) oder einem anderen geeigneten, transparenten, biokompatiblen, nichtlinear optischen Kristall, wie beispielsweise BBO, KnbO₃ oder LiNbO₃ ausgebildet.

Ein dritter Aspekt der Erfindung betrifft eine Verwendung eines erfindungsgemäßen Mikro- oder Nanoroboters in einem erfindungsgemäßen Verfahren.

Ein vierter Aspekt der Erfindung betrifft eine Messanordnung zur Detektion eines Mikro- oder Nanoroboters, insbesondere zur Durchführung eines erfindungsgemäßen Verfahrens, welche einen innerhalb eines biologischen Gewebes angeordneten Mikro- oder Nanoroboter mit wenigstens einem nichtlinear optischen Kristall als Leitstern, eine Lichtquelle zur Aussendung eines kohärenten Lichtstrahls, ein Strahlteilungselement zum Teilen eines Lichtstrahles, Strahlengangleitelemente zum Leiten der Lichtstrahlen und zum Leiten der Lichtstrahlenbündel, einen nichtlinear optischen Referenzkristall und eine Detektionseinheit zur Detektion des Interferenzmusters der überlagerten durch den nichtlinear optischen Kristall des Mikro- oder Nanoroboters und den Referenzkristall erzeugten Messstrahlenbündel sowie eine Detektionseinheit zur Detektion des Intensitätsmusters aufweist. Es kann hierbei eine Detektionseinheit zur Detektion sowohl des Interferenzmusters als auch des Intensitätsmusters eingesetzt werden. Diese Detektionseinheit ist dann in ihrer Position veränderlich, beispielsweise verschiebbar, ausgebildet. Alternativ können zwei Detektionseinheiten an der Messanordnung angeordnet sein.

Die Messanordnung muss dabei derart ausgebildet sein, dass die einzelnen Elemente nach der Art eines Interferometers angeordnet sind, welches digital optische Holographie erlaubt.

Nach einer vorteilhaften Variante der Messanordnung weist die Messanordnung außerdem einen Wellenfrontmodulator auf.

Bevorzugt weist die Messanordnung weiterhin einen spektralen Filter auf.

Der Wellenfrontmodulator ist bevorzugt aus einem Flüssigkristall-Display, wie Liquid Crystal on Silicon (LCoS), oder als ein ferroelektrischer Wellenfrontmodulator oder als ein Mechanical Electrical Micro System (MEMS)-Wellenfrontmodulator ausgebildet.

Ein fünfter Aspekt der Erfindung betrifft ein Computerprogrammprodukt zur Ausführung einer Positionsbestimmung eines Mikro- oder Nanoroboters in biologischem Gewebe, umfassend Befehle, die bewirken, dass die erfindungsgemäße Messanordnung ein erfindungsgemäßes Verfahren ausführt. Bevorzugt erfolgt bei dem Erkennen der Übereinstimmung der Position des Mikro- oder Nanoroboters mit einer festgelegten Zielposition eine induzierte Erwärmung des Mikro- oder Nanoroboters. Besonders bevorzugt erfolgt bei dem Erkennen der Übereinstimmung der Position des Mikro- oder Nanoroboters mit einer festgelegten Zielposition eine Freisetzung eines Stoffes aufgrund einer induzierten Erwärmung des Mikro- oder Nanoroboters.

Ein sechster Aspekt der Erfindung betrifft ein Datenverarbeitungssystem zur Durchführung eines erfindungsgemäßen Verfahrens.

Die Erfindung bietet zahlreiche Vorteile bei der Nutzung von Mikro- oder Nanorobotern in biologischem Gewebe. Eine gezielte Verabreichung von Medikamenten mittels Mikro- oder Nanorobotern kann zu einer Therapie mit verringerten Nebenwirkungen führen. Für die Navigation im biologischen Gewebe wird aber eine externe Bildgebungsmethode benötigt, die eine hohe Ortsauflösung bietet und echtzeitfähig ist. Während optische Messverfahren hohe spatiotemporale Auflösungen ermöglichen, liegt die Herausforderung vor, dass die erreichbare Messtiefe in biologischem Gewebe durch die Streuung von Licht erheblich limitiert ist.

Durch die Integration von nichtlinear optischen Kristallen in Mikro- oder Nanorobotern wird erreicht, dass ein kohärenter, spektral separierter Leitstern vorliegt, mit dem eine echtzeitfähige holographische Wellenfrontformung ermöglicht wird. Mit dieser können die Streuprozesse für einen Punkt korrigiert werden. Damit ist es möglich die Position des Mikro- oder Nanoroboters in tiefem Gewebe zu bestimmen, oder den Mikro- oder Nanoroboter aufzuheizen und damit beispielsweise eine Zellablation durchzuführen.

Dabei wird hier digital optische Holographie genutzt, um ein Beugungsmuster zu erhalten, wobei die Korrektur der Phasenverschiebung dadurch erreicht wird, dass die Phase invertiert wird. Dabei wird der Prozess der digital-optischen Phasenkonjugation verwendet. Dadurch werden die aus dem Interferenzmuster ermittelten, aufgrund der Streueffekte auftretenden Phasenverschiebungen korrigiert.

Mit der hier vorgeschlagenen Methode kann eine Detektion bis in 1 mm, bevorzugt in 10 mm, tiefes Gewebe erreicht werden. Dazu ist eine Kombination von nichtlinearen Kristallen hoher Effizienz mit einer statistischen Auswertung der Messergebnisse erforderlich. Eine Drehung des Mikro- oder Nanoroboters in Verbindung mit einer Auswertung einer bestimmten Frequenz (Lock-In-Technik) verbessert das Signal-Rausch-Verhältnis weiter. Vorteilhaft kann eine closed loop-Strategie unter Einbeziehung der einzelnen Elemente der Positionsbestimmung, der Modulation des Mikro- oder Nanoroboters sowie einer Lock-in-Auswertung erfolgen.

Die mittels der beschriebenen Verfahren, Mikro- oder Nanoroboter und Messanordnungen erreichten Tiefen ermöglichen ganz andere Anwendungen in Geweben, wobei aufgrund der Geschwindigkeit der Messung sogar Anwendungen in sich bewegenden, biologischen Geweben denkbar sind. Die Erfindung erreicht somit nicht nur eine quantitative Verbesserung der Tiefe, sondern es ist aufgrund der Vielzahl der Vorteile eine qualitativ verbesserte Methode zur Positionsbestimmung, zum Tracking sowie bei der Manipulation, also auch der gezielten Erwärmung oder Positionsveränderung, des Mikro- oder Nanoroboters möglich.

Insbesondere kann die neuartige, nichtlineare holographische Bildverarbeitungstechnik für eine simultane Lokalisierung und Abbildung (SLAM) sowie auch für eine Lasermaterialbearbeitung genutzt werden, bei der die Aufheizung des Mikro- oder Nanoroboters zur Bakterienbeseitigung erfolgt. Vorteilhaft kann eine Trajektorie des Mikro- oder Nanoroboters aufgenommen und dargestellt werden.

Konzeptionsgemäß wird bei dem Verfahren zur Positionsbestimmung eines Mikro- oder Nanoroboters in einem biologischen Gewebe, ein an dem Mikro- oder Nanoroboter angeordneter nichtlinear optischer Kristall als Leitstern verwendet. Dabei wird das erzeugte, kohärente Licht halber Wellenlänge ausgewertet, wobei insbesondere die durch die Streuung im biologischen Gewebe resultierende Phasenverschiebung berücksichtigt wird. Die Streuinformationen können somit aufgrund der Kohärenz des erzeugten Lichtes berücksichtigt werden, was eine Fokussierung auf den nichtlinear optischen Kristall erlaubt. Außerdem kann eine spektrale Separation vom Hintergrundlicht ermöglicht werden. Es sind dabei sowohl räumlich als auch zeitlich hohe Auflösungen möglich.

### Ausführungsbeispiele

Nachfolgend soll die Erfindung anhand eines Ausführungsbeispiels eingehender erläutert werden. Das Ausführungsbeispiel bezieht sich auf eine in-vitro und eine in-vivo Messanordnung, welche zur Durchführung des Verfahrens geeignet ist, und soll dabei die Erfindung beschreiben ohne diese zu beschränken.

Anhand von Zeichnungen wird die Erfindung näher erläutert. Dabei zeigen
**Fig. 1** eine "in vitro"-Anordnung zur Aufnahme einer Phasenmaske,
**Fig. 2** eine "in vitro"-Anordnung zur Positionsbestimmung unter Verwendung eines Wellenfrontmodulators,
**Fig. 3** einen "in vivo"-Anordnung zur Aufnahme einer Phasenmaske und
**Fig. 4** eine "in vivo"-Anordnung zur Positionsbestimmung mittels eines Wellenfrontmodulators.

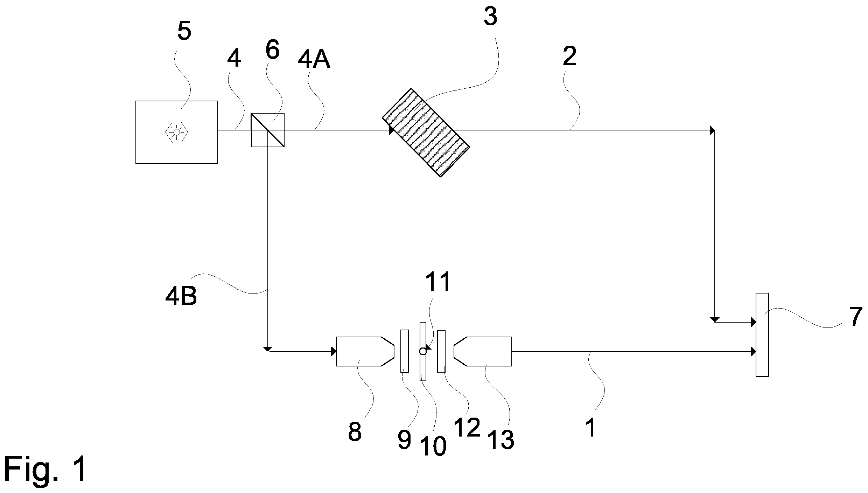

**Figur 1** zeigt eine schematische Darstellung einer "in vitro"-Anordnung zur Aufnahme eines überlagerten Signals aus einem Messstrahlenbündel 1 und einem Referenzstrahlenbündel 2 unter Laborbedingungen. Das Referenzstrahlenbündel 2 wird dabei mittels eines Referenzkristalls 3 erzeugt. Dabei ist der verwendete Referenzkristall 3 ein nichtlinear optischer Kristall, wie beispielsweise BBO, welcher durch einen Referenzanregungsstrahl 4A der Wellenlänge λ angeregt wird. Dafür stellt ein Laser 5 einen Anregungsstrahl 4 zur Verfügung, welcher mittels eines Strahlteilers 6 in einen Referenzanregungsstrahl 4A und einen Messanregungsstrahl 4B geteilt wird. Das durch den Referenzkristall 3 erzeugte Referenzstrahlenbündel 2 wird auf eine Detektionseinheit 7, wie beispielsweise eine sCMOS-Kamera, geleitet.

Der Messanregungsstrahl 4B wird über ein erstes Objektiv 8 durch ein erstes Gewebe 9 auf eine Probe 10 geleitet. An dieser Probe 10 ist ein Messkristall 11 angeordnet. Der Messkristall 11 ist ein nichtlinear optischer Kristall, wie beispielsweise BTO. Durch den Messanregungsstrahl 4B, welcher mittels des Objektivs 8 durch das erste Gewebe 9 auf die Probe 10 fokussiert wird, wird im Messkristall 11 das Signal der zweiten Harmonischen erzeugt. Das im Messkristall 11 induzierte Signal der zweiten Harmonischen wird als Messstrahlenbündel 1 durch ein zweites Gewebe 12 über ein zweites Objektiv 13 auf die Detektionseinheit 7 geleitet.

Eine Auswertung der Signale des Messstrahlenbündels 1 sowie des Referenzstrahlenbündels 2 ermöglichen die Aufnahme eines zweidimensionalen Interferenzmusters sowie die Ermittlung eines zweidimensionalen Phasenverschiebungsmusters.

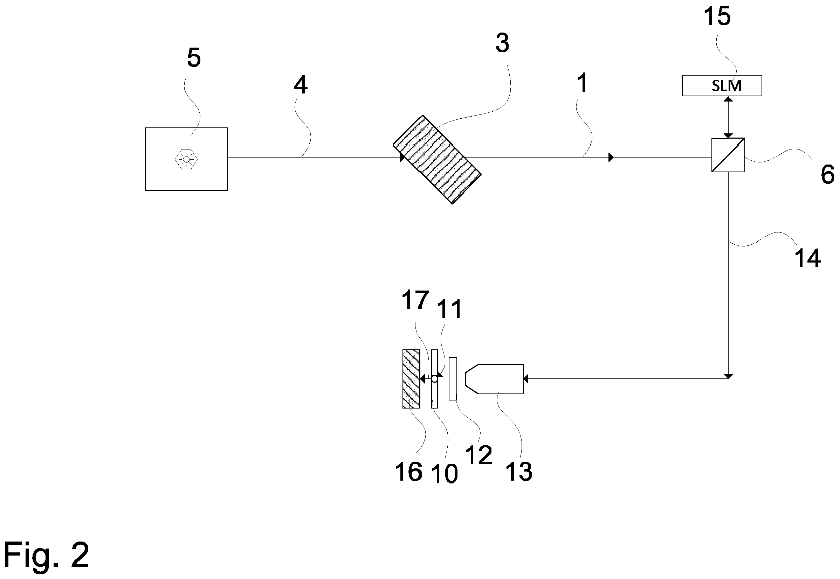

**Figur 2** zeigt eine schematische Darstellung einer "in vitro"-Anordnung zur Fokussierung eines korrigierten kohärenten Messstrahlenbündels 14 auf eine Probe 10 mit einem Messkristall 11. Dafür wird ein kohärenter Anregungsstrahl 4, welcher durch einen Laser 5 zur Verfügung gestellt wird, auf den ursprünglich als Referenzkristall 3 verwendeten nichtlinear optischen Kristall 3 geleitet, wo der kohärente Anregungsstrahl 4 eine zweite Harmonischen induziert. Die im Referenzkristall 3 induzierte zweite Harmonische wird in dieser Anordnung als Messstrahlenbündel 1 auf einen Wellenfrontmodulator 15 geleitet, wobei hier ein Strahlteiler 6 zum Einsatz kommt. Das durch den Wellenfrontmodulator 15 korrigierte Messstrahlenbündel 14 wird unter Einsatz eines zweiten Objektivs 13 durch das zweite Gewebe 12 auf die Probe 10 fokussiert. Der in der Probe 10 angeordnete nichtlineare optische Kristall 11 erzeugt aus dem korrigierten Messstrahlenbündel 14 eine zweite Harmonische, deren Signal mittels einer zweiten Detektionseinheit 16 aufgenommen wird.

Es wird in dieser Anordnung somit aus dem kohärenten Licht des Anregungsstrahls 4 mit der Wellenlänge λ ein kohärentes Messstrahlenbündel 1 der Wellenlänge λ/2 erzeugt. Dieses Messstrahlenbündel 1 wird nun unter Verwendung des ausgewerteten zweidimensionalen Interferenzmusters, welches mittels der Anordnung aus Figur 1 ermittelt wurde, durch den Wellenfrontmodulator 15 derart korrigiert, dass ein korrigiertes Messstrahlenbündel 14 der Wellenlänge λ/2 mittels eines Objektivs trotz der Streuung durch ein Gewebe 12 auf eine Probe 10 fokussiert werden kann. Somit kann aufgrund der Korrektur durch den Wellenfrontmodulator 15 eine gute Fokussierung auf den nichtlinear optischen Kristall 11 erfolgen. Das Signal der durch den nichtlinear optischen Kristall 11 erzeugten zweiten Harmonischen 17 der Wellenlänge λ/4 kann mittels der zweiten Detektionseinheit 16 aufgenommen werden.

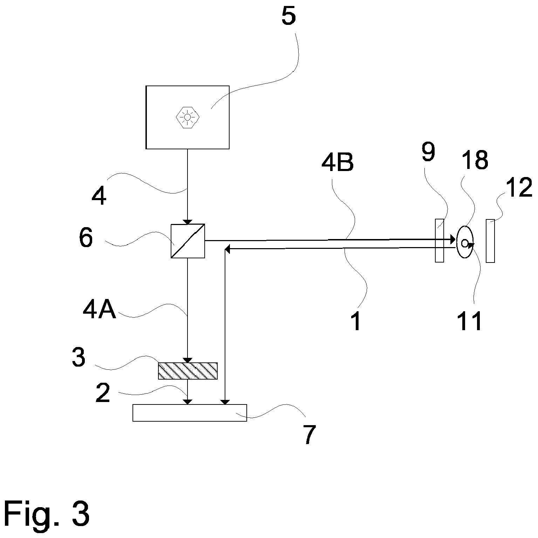

**Figur 3** zeigt eine schematische Darstellung einer "in vivo"-Anordnung, welche eine realisierbare Möglichkeit für eine Anwendung der Technologie in einem Mikro-oder Nanoroboter darstellt. Ein Laser 5 stellt dabei einen kohärenten Anregungsstrahl 4 der Wellenlänge λ zur Verfügung, welcher auf einen Strahlteiler 6 geleitet wird, wo er in einen Referenzanregungsstrahl 4A und einen Messanregungsstrahl 4B geteilt wird. Eine geeignete Wellenlänge für den Anregungsstrahl ist λ = 1,6 µm. Der Referenzanregungsstrahl 4A wird auf einen Referenzkristall 3 mit den Eigenschaften eines nichtlinear optischen Kristalls geleitet, in dem eine zweite Harmonische der Wellenlänge λ/2 = 0,8 µm, erzeugt wird, deren Signal als kohärentes Referenzstrahlenbündel 2 mittels der Detektionseinheit 7 aufgenommen wird.

Der Messanregungsstrahl 4B wird durch ein erstes Gewebe 9, auf einen Mikro- oder Nanoroboter 18 geleitet, welcher einen nichtlinear optischen Messkristall 11 aufweist. Beim Durchqueren des ersten Gewebes 9 wird der Messanregungsstrahl 4B gestreut und trifft daraufhin auf einem Mikro- oder Nanoroboter 18, welche einen nichtlinear optischen Messkristall 11 aufweist. In dem nichtlinear optischen Messkristall 11 wird eine zweite Harmonische erzeugt, welche als Messstrahlenbündel 1 ebenfalls zur Detektionseinheit 7 geleitet wird. Die Detektionseinheit 7 nimmt das Interferenzmuster aus Messstrahlenbündel 1 und Referenzstrahlenbündel 2 auf, woraus ein zweidimensionales Phasenverschiebungsmuster ermittelt werden kann.

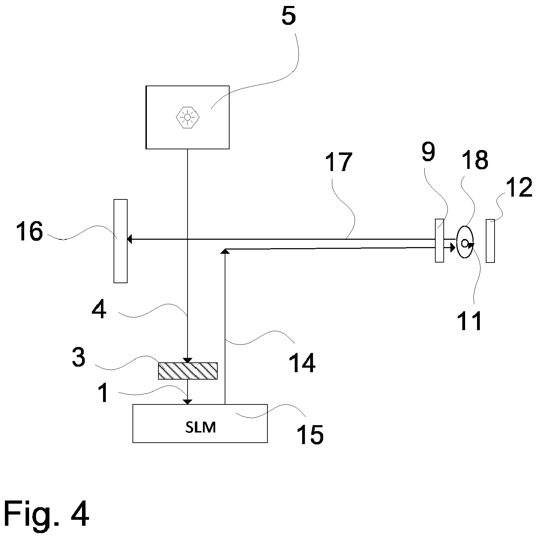

**Figur 4** zeigt eine schematische Darstellung einer "in vivo"-Anordnung zur Positionsbestimmung eines Mikro- oder Nanoroboters 18 mit einem durch einen Wellenfrontmodulator 15 korrigierten Messstrahlenbündel 14. Ein durch einen Laser 5 erzeugter, kohärenter Anregungsstrahl 4 der Wellenlänge λ = 1,6 µm wird auf den Referenzkristall 3 geleitet und erzeugt dort eine zweite Harmonische. Die zweite Harmonische des Referenzkristalls 3 wird hier als Messstrahlenbündel 1 der Wellenlänge λ/2 = 0,8 µm auf einen Wellenfrontmodulator 15 geleitet. Das durch den Wellenfrontmodulator korrigierte Messstrahlenbündel 14 durch ein erstes Gewebe 9 geleitet und trifft dann auf den Mikro- oder Nanoroboter 18. Aufgrund der durch den Wellenfrontmodulator 15 erfolgten Korrektur in Form einer Veränderung der Phase des kohärenten Lichtes kann das korrigierte Messstrahlenbündel 14 auf den Messkristall 11 fokussiert werden. Die durch den Wellenfrontmodulator erfolgte Phasenverschiebung entspricht hier einer Phaseninversion, sodass die Streueffekte im Gewebe 9 dadurch ausgeglichen werden können. Es erfolgt hier eine gezielte Anregung des nichtlinear optischen Messkristalls 11, wodurch in dem Messkristall 11 eine zweite Harmonische 17 mit der Wellenlänge λ/4 erzeugt wird. Das Signal der durch den Messkristall 11 erzeugten zweiten Harmonischen 17 wird mittels eine Detektionseinheit 16 aufgezeichnet. Dieses Signal kann genutzt werden, um eine Positionsbestimmung des Messkristalls 11 und somit des Mikro- oder Nanoroboters 18 zu realisieren.

Es liegt dabei nur ein optischer Zugang vor, womit der Mikro- oder Nanoroboter 18 außerhalb des biologischen Gewebes, welches als erstes Gewebe 9 dargestellt ist, detektiert und vermessen werden kann. Mittels der digitalen harmonischen Holographie werden Informationen ermittelt, welche für das frequenzverdoppeltes Licht, also jenes der halben Wellenlänge λ/2, mit dem Wellenfrontmodulator 15 korrigiert werden. Dieses aberrationskorrigierte Licht, welches als korrigiertes Messstrahlenbündel 14 durch die Anordnung geleitet wird, wird mittels der Detektionseinheit 16, welche beispielsweise eine sCMOS-Kamera ist, aufgenommen. Dadurch erfolgt eine dreidimensionale Positionsmessung des Mikro- oder Nanoroboters 18 innerhalb von tiefem biologischen Gewebe.

### Zitierte Nichtpatentliteratur:

[1] "Real-Time IR Tracking of Single Reflective Micromotors through Scattering Tissues", Azaam Aziz, Mariana Medina-Sänchez, Nektarios Koukourakis, Jiawei Wang, Robert Kuschmierz, Hannes Radner, Jürgen W. Czarske, and Oliver G. Schmidt, Adv. Funct. Mater. 2019, 29, 1905272
[2] "Imaging with second-harmonic radiation probes in living tissue!, Grange et.al., Biomedical Optics Express 2532, 1 September 2011, Vol. 2, No. 9
[3] "Seeing through turbidity with harmonic holography", Pu et al., Applied Optics, Vol. 52, No. 4
[4] "Imaging with second-harmonic radiation probes in living tissue", Rachel Grange, Thomas Lanvin, Chia-Lung Hsieh, Ye Pu, und Demetri Psaltis, Vol. 2, No. 9 / BIOMEDICAL OPTICS EXPRESS 2532, 2011
[5] "Field-Programmable System-on-Chip-Based Control System for Real-Time Distortion Correction in Optical Imaging", H Radner, J Stange, L Büttner, J Czarske, IEEE Transactions on Industrial Electronics 68 (4), 3370-3379, 2020)
[6] "Spiral phase mask shadow-imaging for 3D-measurement of flow fields", M Mattern, J Sturm, L Büttner, JW Czarske - Optics express, 2016 - osapublishing.org
[7] Forouhesh Tehrani K, Koukourakis N, Czarske J, Mortensen LJ. "In situ measurement of the isoplanatic patch for imaging through intact bone", Journal of Biophotonics. 2021; 14: e202000160
[8] "Focusing light through scattering tissue", N. Koukourakis, J. Czarske, Research Project URL: https://tu-dresden.de/ing/elektrotechnik/iee/biolas/research/deep-tissue-applications
[9] "Photoacoustic Imaging-Trackable Magnetic Microswimmers for Pathogenic Bacterial Infection Treatment", Lisi Xie, Xin Pang, Xiaohui Yan*, Qixuan Dai, Huirong Lin, Jing Ye, Yi Cheng, Qingliang Zhao, Xing Ma, Xianzhong Zhang, Gang Liu, and Xiaoyuan Chen, ACS Nano 2020, 14, 3, 2880-2893

### Bezugszeichen

- 1: Messstrahlenbündel
- 2: Referenzstrahlenbündel
- 3: Referenzkristall, nichtlinear optischer Kristall
- 4: Anregungsstrahl, kohärenter Anregungsstrahl, kohärenter Lichtstrahl
- 4A: Referenzanregungsstrahl, Referenzstrahl
- 4B: Messanregungsstrahl, Messstrahl
- 5: Laser, Lichtquelle
- 6: Strahlteiler
- 7: erste Detektionseinheit, erste Kamera, erste sCMOS-Kamera
- 8: Erstes Objektiv
- 9: Erstes Gewebe nun
- 10: Probe
- 11: Messkristall, nichtlinearer optischer Kristall
- 12: Zweites Gewebe, zweite Gewebeprobe
- 13: Zweites Objektiv
- 14: Korrigiertes Messstrahlenbündel, korrigiertes kohärentes Messstrahlenbündel
- 15: Wellenfrontmodulator, SLM
- 16: Zweite Detektionseinheit, zweite Kamera, zweite sCMOS-Kamera
- 17: Zweite Harmonische des Messkristalls
- 18: Mikro- oder Nanoroboter

## Patentansprüche

1. Verfahren zur Positionsbestimmung von Mikro- oder Nanorobotern (18) in einem biologischen Gewebe (9, 12), folgende Schritte umfassend:
**1)** Aussenden eines gepulsten Laserstrahls (4) mit einer Wellenlänge λ
**2)** Teilen des gepulsten Laserstrahls (4) in einen kohärenten Messstrahl (4B) der Wellenlänge λ und einen kohärenten Referenzstrahl (4A) der Wellenlänge λ
**3)** Leiten des kohärenten Messstrahles (4B) auf einen in dem biologischen Gewebe (9) angeordneten Mikro- oder Nanoroboter (18), wobei der Mikro- oder Nanoroboter (18) wenigstens einen nichtlinear optischen Kristall (11) aufweist, und Leiten des kohärenten Referenzstrahles (4A) auf einen nichtlinear optischen Referenzkristall (3)
**4)** Überlagerung des durch den nichtlinear optischen Referenzkristall (3) unter Erzeugung der zweiten Harmonischen emittierten, kohärenten Referenzstrahlbündels (2) der Wellenlänge λ/2 mit dem durch den nichtlinear optischen Kristall (11) des Mikro- oder Nanoroboters (18) unter Erzeugung der zweiten Harmonischen emittierten, kohärenten Messstrahlbündels (1) der Wellenlänge λ/2
**5)** Detektion des zweidimensionalen Interferenzmusters der Überlagerung aus dem Referenzstrahlbündel (2) und dem Messstrahlbündel (1) mittels einer ersten Detektionseinheit (7),
**6)** Ermittlung der Position des Mikro- oder Nanoroboters (18).

2. Verfahren nach Anspruch 1, **dadurch gekennzeichnet, dass** die Ermittlung der Position nach Schritt 6) folgende Schritte umfasst:
6.1) Ermittlung eines zweidimensionalen inversen Phasenverschiebungsmusters aus dem zweidimensionalen Interferenzmuster mittels digital optischer Holographie
6.2) Leiten eines kohärenten Messstrahlenbündels (1) auf einen das inverse Phasenverschiebungsmuster aufweisenden Wellenfrontmodulator (15), wodurch das kohärente Messstrahlenbündel (1) eine Korrektur in Form einer digital-optischen Phasenkonjugation erfährt
6.3) Leiten des korrigierten Messstrahlenbündels (14) auf den Mikro- oder Nanoroboter (18) mit dem nichtlinear optischen Kristall (11)
6.4) Aufnahme eines Intensitätsmusters des durch den Mikro- oder Nanoroboter (18) reflektierten Lichts mittels einer zweiten Detektionseinheit (16)
6.5) Bestimmung der lateralen Position des Mikro- oder Nanoroboters (18) aus dem aufgenommenen Intensitätsmuster

3. Verfahren nach Anspruch 2, **dadurch gekennzeichnet, dass** mittels einer Regelung die Ermittlung des inversen Phasenverschiebungsmusters nach Schritt 6.1) und die Wellenfrontformung des kohärenten Lichtstrahles (4) mittels des Wellenfrontmodulators (15) als selbstlernendes System ausgebildet sind.

4. Verfahren nach einem der Ansprüche 2 oder 3, **dadurch gekennzeichnet, dass** in Schritt 6.2) ein durch den Referenzkristall (3) ausgesandtes, kohärentes Lichtstrahlenbündel (1) der Wellenlänge λ/2 verwendet wird und dass das Signal des durch den nichtlinear optischen Kristall (11) elastisch gestreuten Lichtstrahlbündels der Wellenlänge λ/2 detektiert wird.

5. Verfahren nach Anspruch 1, **dadurch gekennzeichnet, dass** die Schritte 3) bis 5) mehrmals nacheinander durchgeführt werden, wobei der kohärente Messstrahl (4B) in Schritt 3) jeweils in einem unterschiedlichen, eingestellten Einfallswinkel auf den nichtlinear-optischen Kristall (11) des Mikro- oder Nanoroboters (18) fällt und die Ermittlung der Position nach Schritt 6) unter Auswertung unterschiedlicher aus den Interferenzmustern ermittelter Phasenverschiebungsmuster mittels neuronaler Netze erfolgt.

6. Verfahren nach Anspruch 6, **dadurch gekennzeichnet, dass** die Einstellung der Einfallswinkel dadurch realisiert wird, dass der kohärente Messstrahl (4B) durch einen zeitlich modulierten Wellenfrontmodulator (15) oder einen rotierenden Spiegel in seinem Verlauf beeinflusst wird.

7. Verfahren nach einem der Ansprüche 1 bis 7, **dadurch gekennzeichnet, dass** ferner die Bestimmung der Tiefe der Position des Mikro- oder Nanoroboters (18) erfolgt.

8. Verfahren nach einem der Ansprüche 1 bis 7, **dadurch gekennzeichnet, dass** der Mikro- oder Nanoroboter (18) mittels eines externen magnetischen Feldes in Rotation mit einer Rotationsfrequenz versetzt wird, sodass das in Schritt 5) detektierte, auszuwertende Interferenzmuster mit der Rotationsfrequenz oder mit der doppelten Rotationsfrequenz moduliert ist.

9. Verfahren nach einem der Ansprüche 1 bis 8, **dadurch gekennzeichnet, dass** folgender Verfahrensschritt erfolgt: der korrigierte Messstrahl oder das korrigierte Messstrahlenbündel (14) gemäß Schritt 6.3) wird auf den Mikro- oder Nanoroboter (18) fokussiert, sodass eine gezielte Steuerung der Temperatur des Mikro- oder Nanoroboters (18) durch eine Variation der Intensität des auf den Mikro- oder Nanoroboter (18) fokussierten korrigierten Messstrahles oder korrigierten Messstrahlenbündels (14) erfolgt.

10. Verfahren nach Anspruch 9, **dadurch gekennzeichnet, dass** eine Erwärmung von Metallpartikeln im nichtlinear optischen Kristall erfolgt.

11. Mikro- oder Nanoroboter (18) zur Anwendung in biologischem Gewebe, aufweisend ein Aktormaterialelement als Angriffspunkt für eine Kraft zur Bewegung des Mikro- oder Nanoroboters (18), **dadurch gekennzeichnet, dass** der Mikro- oder Nanoroboter (18) einen nichtlinear optischen Kristall (11) als Leitstern aufweist.

12. Mikro- oder Nanoroboter (18) nach Anspruch 11, **dadurch gekennzeichnet, dass** der Mikro-oder Nanoroboter (18) ein aus einem magnetischen oder einem ferromagnetischen Material ausgebildetes Magnetfeldangriffselement aufweist.

13. Verwendung eines Mikro- oder Nanoroboters (18) nach Anspruch 11 oder 10 zur Durchführung eines Verfahrens nach einem der Ansprüche 1 bis 10.

14. Messanordnung zur Durchführung eines Verfahrens nach einem der Ansprüche 1 bis 10, aufweisend einen innerhalb eines biologischen Gewebes angeordneten Mikro- oder Nanoroboter (18) mit wenigstens einem nichtlinear optischen Kristall (11) als Leitstern, eine Lichtquelle (5) zur Aussendung eines gepulsten, kohärenten Lichtstrahls (4), ein Strahlteilungselement (6) zum Teilen des kohärenten Lichtstrahles (4), Strahlengangleitelemente zum Leiten der Lichtstrahlen (4, 4A, 4B) und der Lichtstrahlenbündel (1, 2, 14), einen nichtlinear optischen Referenzkristall (3) und eine erste Detektionseinheit (7) zur Detektion des Interferenzmusters der überlagerten durch den nichtlinear optischen Kristall (11) und den Referenzkristall (3) erzeugten Messstrahlenbündel (1, 2) und eine zweite Detektionseinheit (16) zur Detektion des Intensitätsmusters.

15. Computerprogrammprodukt zur Ausführung einer Positionsbestimmung eines Mikro- oder Nanoroboters (18) in biologischem Gewebe, umfassend Befehle, die bewirken, dass die Messanordnung nach Anspruch 14 ein Verfahren nach einem der Ansprüche 1 bis 10 ausführt.

16. Computerprogrammprodukt nach Anspruch 15, **dadurch gekennzeichnet, dass** bei dem Erkennen der Übereinstimmung der Position des Mikro- oder Nanoroboters (18) mit einer festgelegten Zielposition eine induzierte Erwärmung des Mikro- oder Nanoroboters (18) erfolgt.

17. Datenverarbeitungssystem umfassend Mittel zur Durchführung eines der Verfahren gemäß der Ansprüche 1 bis 10.
